# EUROPEAN PATENT APPLICATION

(11) **EP 4 517 764 A1**
(43) Date of publication of application: **05.03.2025**
(21) Application number: 23194816.7
(22) Date of filing: 01.09.2023
(51) Int. Cl.: G16H 10/60, G16H 30/20, G16H 40/63

(54) **AUGMENTING MEDICAL DATA**

(71) Applicant: Koninklijke Philips N.V., 5656 AG Eindhoven (NL)
(72) Inventor: SERLIE, Iwo, Eindhoven (NL); CUPPEN, Roel, Eindhoven (NL); BULUT, Murtaza, 5656AG Eindhoven (NL); BOUWMAN, Wilbert, Eindhoven (NL); RASMIJN, Anita, Eindhoven (NL)
(74) Representative: Philips Intellectual Property & Standards

(57) **Abstract**

Proposed concepts aim to provide schemes, solutions, concepts, designs, methods, and systems pertaining to medical data augmentation. In particular, medical data is augmented (e.g., linked to or annotated with) generation distance data describing a distance between user(s) and the medical data generation device during generation of the medical data. Subsequently, at least a part of the medical data shown to viewer(s) of the medical data may be determined based on the generation distance data. Accordingly, appropriate medical data may be presented to the viewer without the need for manual configuration. That is, based on observations of the distance of users(s) (e.g., healthcare professionals) who interact with the data generator, and user(s) who watch or are close to the data generators, it is possible to anticipate what parts of the medical data may be needed for subsequent viewing by a user.

## Description

### FIELD OF THE INVENTION

The present invention relates to medical data augmentation, and also to providing said medical data.

### BACKGROUND OF THE INVENTION

Healthcare professionals need to receive and provide the right information at the right time. The information is usually part of an electronic medical record (EMR) associated with a single patient, which contains huge amounts of data. Systems that support the selection of the correct data subsets whilst avoiding the need for manual input contribute to a more productive environment.

EMR systems are typically user profile based, which means that they make it possible to configure specific views presented to users of different roles. Accordingly, these systems may display only the most relevant patient information for the role of the viewer. For example, based on the view's authorization level, the system may determine which information and functionalities are required for their role or specialism.

However, such traditional role-based data management systems struggle to fluidly follow alternative collaboration patterns that were not envisioned when the software system was designed, implemented, and/or configured. That is, role based patterns to deliver data often do not suffice in dynamic environments where healthcare professionals with different backgrounds and roles support each other in the execution of protocols.

Therefore, there exists a need for means that automatically provide relevant information without the need for manual configuration.

### SUMMARY OF THE INVENTION

The invention is defined by the claims.

According to examples in accordance with an aspect of the invention, there is provided a method of generating augmented medical data. The method comprises:
obtaining medical data generated by a medical data generation device;
obtaining generation distance data describing a distance between one or more users and the medical data generation device during generation of the obtained medical data; and
augmenting the obtained medical data based on the obtained generation distance data.

It has been realised that a particularly useful piece of information for understanding the context of the generation of medical data is data describing a distance between one or more users and the medical data generation device (i.e. software or hardware configured for the generation of medical data). For example, by augmenting (i.e. linking or adding) generation distance data to the medical data, (parts of) the medical data may be appropriately presented to a viewer. Additionally, the distance information may be useful for other purposes, such as tracking progression of an infection in a healthcare setting (e.g. a close proximity to a data generator may lead to a higher chance of infection spread event).

For example, the distance of a certain user to the medical data generation device may provide valuable information that enables the natural progression of a workflow. That is, if it is known that a user was close to a data generation device during medical data generation, then access to said medical data may be made simpler or faster (e.g., presented automatically, require fewer inputs/clicks/human-machine interactions) to access by the user when they are in the proximity of a data viewer. Conversely, if a user was distant from the data generation device, then access to the medical data may not be simplified (i.e. not or less likely to be presented automatically) for the user when in proximity of the data viewer (e.g., to ensure that the information presented is relevant to the user, or even for privacy considerations). Of course, in certain circumstances the converse may be beneficial, with medical data information shown automatically to a viewing user as a priority if they were not near the data generation device during generation, so that they may quickly be aware of the results.

In any case, the generation distance data may be used to simplify and expedite access to and presentation of the medical data for certain users. Nevertheless, it should be understood that the precise usage of the generation distance data may vary depending on the circumstances, depending on user/healthcare provider preferences and the context of the medical data.

Furthermore, if it is indicated by the generation distance information that no users were in close proximity to the medical data generator during generation of the medical data, this may aid in the assessment of whether the medical data can be deemed reliable or not. This may be particularly useful in scenarios where there are brief anomalies that could either be explained by equipment malfunction or incorrect generation techniques (noticeable by a user if in close proximity to the medical data generation), or could instead indicate a certain pathology. In any case, the augmentation of the medical data with generation distance data enables a more thorough understanding of the medical data, whether for interpreting the medical data or for appropriate presentation of the medical data.

Accordingly, it is beneficial to ensure that generation distance information is used to augment medical data, to enable appropriate utilisation of the medical data post-generation. Indeed, this potential benefit increases as medical environments become ever-more collaborative between different users having different and changeable roles, teams and expertise.

In some embodiments, the method may further comprise obtaining user characteristic data describing at least one characteristic of the one or more users, and augmenting the obtained medical data based on the obtained user characteristic data.

In order to further exemplify the benefit of the invention, obtaining and storing information regarding the type of user may be particularly useful. By augmenting the medical data with such information an improved understanding of the medical data may be achieved, both for future interpretation and determination of appropriate presenting/provision for viewing.

For example, if a cardiologist is known to be the nearest user during generation of the medical data, then the medical data may be predicted with high certainty to be associated with cardiology. Thus, when the same or different cardiologist approaches a device configured for outputting the medical data, it may be known that said medical data is likely to be relevant to them, such that it is presented and/or highlighted. Of course, this may enable improved workflows between users of similar roles picking up work where the last user left. Indeed, this reduces the need for manual user input to ensure that the correct information is presented, saving valuable time and cognitive effort.

Preferably, the user characteristic data may include at least one of a user identifier, a user role, a user action, a user group, a user experience, and a user expertise.

In some cases, obtaining the generation distance data may comprise determining, for each of the one or more users, a value indicative of a distance between the user and the medical data generator.

There may be various ways to determine proximity of the user to the medical data generation device. For example, known absolute positions of the user and data generation device may be leveraged to determine distance. Alternatively, a relative measure (such as a signal strength of a signal transmitted between the data generation device and a user device) may be used to determine the proximity. Of course, the value need not be a precise measure. Instead, the value may indicate a range of distance between the user and data generator (e.g., proximate, same room, outside of room, etc.).

More particularly, determining the value indicative of the distance between the user and the medical data generator may comprise obtaining a location of the user during generation of the obtained medical data, obtaining a location of the medical data generator during generation of the obtained medical data, and generating the value based on the obtained location of the user and the obtained location of the medical data generator.

For example, the location of the one or more users and/or the medical data generator may be obtained from a real time location system.

This may provide an accurate measure of the distance between the user and generation device, and thus provide accurate information for future understanding of the medical data.

Furthermore, the location information obtained from the real time location system may also be added/linked to the medical data. That is, in addition to the distance information, the absolute location information may also be augmented to the user data.

In some embodiments, the method may further comprise comparing the generation distance data to a predetermined requirement. Accordingly, augmenting the obtained medical data based on the obtained distance data may be performed responsive to the generation distance data satisfying the predetermined requirement. Preferably, the predetermined requirement may comprise at least one of a maximum distance, a minimum amount of time within a predetermined distance, and a predefined location requirement.

Put another way, the medical data may only be augmented with the generation distance data should such data meet requirements. The requirements may be set such that the generation distance information is relevant. For example, if a user is not present for the generation of the medical data, then this may be implied by having no generation distance information linked to the medical data for that user. Thus, this feature may ensure that less relevant information is not used to augment the medical data, saving storage space and reducing complexity of interpretation.

Also, augmenting the obtained medical data may comprise linking the generation distance data as metadata to the medical data, and/or annotating the medical data with the generation distance data.

There are various ways in which the medical data may be augmented using the generation distance data. Two such ways are to supply the generation distance data as metadata, or simply annotating the medical data.

According to yet another aspect of the invention, there is provided a method for providing medical data to a viewer. The method comprises:
retrieving augmented medical data generated according to any of the methods outlined above;
determining at least a part of the medical data to be output to the viewer based on the generation distance data.

Accordingly, a key advantage of the invention of providing appropriate (parts of) medical data to a viewer may be achieved by leveraging the generation distance data used to augment the medical data.

The method may further comprise obtaining viewing distance data describing a distance between the viewer and a device adapted to output medical data. In this case determining the medical data to be output to the viewer may be further based on the viewing distance data.

Of course, another relevant aspect to providing appropriate data includes a distance of the viewer to the device that generated the medical data. This can be particularly useful in tandem with the generation distance data with any similarities or differences being useful for the appropriate selection of (parts of) medical data to be output to the viewer.

In some embodiments, the method may also comprise obtaining viewer characteristic data describing at least one characteristic of the viewer. In this case determining the medical data to be output to the viewer may be further based on the obtained viewer characteristic data.

Similarly, information about the viewer may be useful for the appropriate selection of (parts of) medical data to be output to the viewer. This may be particularly true if the characteristics of the user(s) (which may have been added as metadata to the medical data in the medical data generation stage) in the vicinity of the data generation device during generation of the medical data is known.

According to additional examples in accordance with another aspect of the invention there is provided a computer program product comprising computer program code means which, when executed on a computing device having a processing system, cause the processing system to perform all of the steps of at least one of the above outlined embodiments of the invention.

According to examples in accordance with additional aspects of the invention there is provided a system generating augmented medical data, comprising:
an interface configured to obtain medical data generated by a medical data generation device, and obtain generation distance data describing a distance between one or more users and the medical data generation device during generation of the obtained medical data; and
a processor configured to augment the obtained medical data based on the obtained generation distance data.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 presents a block diagram of a system for medical data augmentation according to an illustrative embodiment of the invention;
Fig.2 presents a block diagram of a system for presenting augmented medical data according to another aspect of the illustrative embodiment of the invention;
Fig.3 presents a flow diagram of a method of augmenting medical data according to another aspect of the invention;
Fig.4 presents a flow diagram of a method for providing medical data to a viewer according to a further aspect of the invention;
Fig.5 is a simplified block diagram of a system for augmenting medical data according to yet another embodiment of the invention; and
Fig.6 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

It should be understood that the Figures are merely schematic and are not drawn to scale. It should also be understood that the same reference numerals are used throughout the Figures to indicate the same or similar parts.

Proposed concepts aim to provide schemes, solutions, concepts, designs, methods, and systems pertaining to medical data augmentation. In particular, medical data is augmented (e.g., linked to or annotated with) generation distance data describing a distance between user(s) and the medical data generation device during generation of the medical data. Subsequently, at least a part of the medical data shown to viewer(s) of the medical data may be determined based on the generation distance data. Accordingly, appropriate medical data may be presented to the viewer without the need for manual configuration. That is, based on observations of the distance of user(s) (e.g., healthcare professionals) who interact with the data generator, and user(s) who watch or are close to the data generators, it is possible to anticipate what parts of the medical data may be needed for subsequent viewing by a user.

The proposed invention enables the automatic provision of relevant information without the need for configuration or time-consuming manual interaction with medical data (e.g., electronic medical records). It has been realised that, by observing a distance between users that interact with the system that generates data (i.e., the medical data generation device) as well as nearby users, the parts of the medical data that may be needed for a specific user may be determined.

Accordingly, the medical data augmented based on generation distance data enables the determination of the correct/appropriate medical data to be presented to users (e.g., healthcare workers) who collaborate in a dynamic environment. For example, consider healthcare workers who take over work at a specific machine in order to free-up time of another clinical specialist.

In other words, it is proposed to automatically capture the user(s) working at a specific device (hardware or software) or patient, which are both considered medical data generation devices, and to include an estimated physical distance of user(s) to these medical data generation devices. The physical distance (i.e. generation distance data) of user(s) to a data generator may be persisted as meta data with the generated medical data.

When displaying medical data to a second user, the recorded proximity of generation of medical data to a first user and/or patient are used to associate that data to a first and second user. Associations may be based on a user identifier, as well as role or team identifier. For example, the association can be established, if a user has been near a medical data generation device for a given minimum period of time. When including the role, any user with identical or similar roles may be associated with the generated medical data. The proximity may be amended with other user characteristics such as role, expertise, team, unit and hospital associated with the user.

As a result, this allows medical data presentation systems to better cope with different users who take over responsibilities (each user having different roles, skills, or experience). That is, this allows appropriate medical data to be presented depending on the user who is viewing the medical data. Also, the medical data may be shared between different parties in a reliable and robust manner. In addition, it facilitates more efficient information sharing among members of the same team by focussing on the correct/appropriate medical data at the right time. For example, given the hand-over work from a first user to a second user, it may be possible to continue the medical dataflow with the second worker without specific configuration.

This may be particularly useful at times when the medical data is generated and observed in a crowded environment (e.g., an emergency department), and it is urgent to utilize available resources in an optimal way. In this case, the proposed invention may cope with continuation of workflow by different users by showing the correct medical data without explicit configuration of workflow based on the captured user proximity to the data generation process.

Furthermore, this further improves data sharing between different hospital units and different hospitals. By way of brief explanation, having the distance information of users added to the medical data, (in case of data sharing), the receiving party/hospital can make use of the distance information to determine a quality of data collection (as a quality measure) and can determine if the data has been collected according to the advised protocols, and by the people with the right speciality and expertise (as a trust measure).

Moreover, the proposed invention may improve the way in which electronic medical records (EMRs) are used to access, visualize and update patient medical data. In other words, the distance information can be used to improve the interaction with EMR systems. For example, the medical data associated with distance data satisfying certain distance criteria can have different display, accessibility, editing, and processing configurations. Specifically, when a user (or similar users, where the similarity can be determined in terms of role, team, associated patient(s), etc.) logs on to the EMR, the medical data having distance information indicative of a user proximate during generation may be made immediately visible and accessible, enabling the user to quickly visualize, check, correct and approve the data. The medical data having distance information indicative of a user far away during generation may be highlighted, enabling the user to become aware of other relevant data.

Also, access information (i.e., viewing information) can be stored, and used to determine how the distance information can be used to modify the interaction with the EMR. For example, first interaction after data collection, can happen as described above ("non-distant" data access made faster and simpler, and "distant" data highlighted), while second interaction with the EMR, can be different, for example, "distant" data made more easily accessible, while "non-distant data" less easily accessible (e.g., requires more clicks, or additional user-computer interactions).

Another advantage of the invention may be appreciated in relation to central monitoring displays/dashboard. Such displays provide medical for a plurality of subjects, for example in one hospital unit. In this case, depending on distance information, the medical data provided by the user interface may be adapted. For example, areas and information corresponding to particular patients or to particular data/information can be displayed in different manner (e.g., most relevant (determined based on distance) display on top, or highlighted), selected information displayed more (or less) prominently, hidden information (which is not typically displayed) becoming visible, and so on.

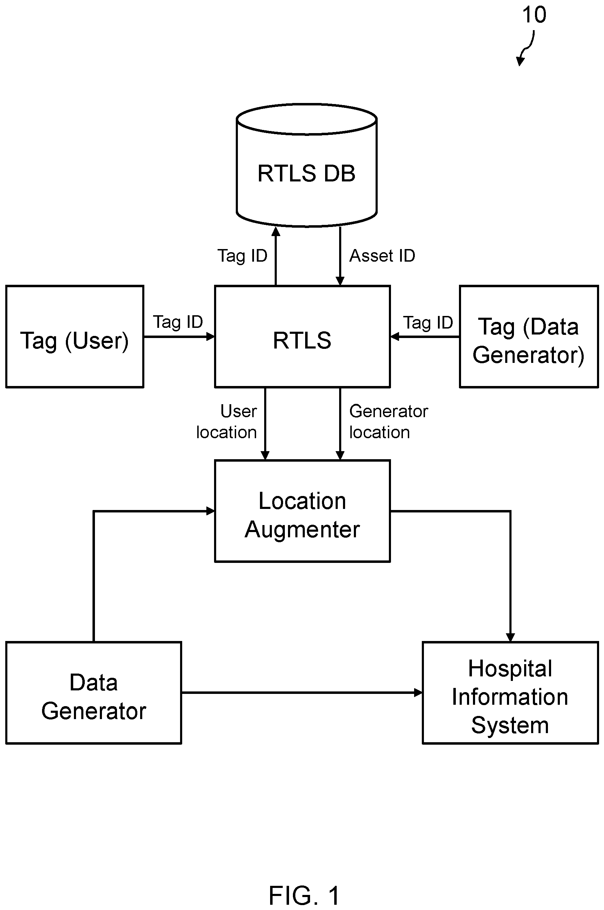

Turning to Fig. 1, there is presented a simplified block diagram of a system that may implement the medical data augmentation according to the core concept of the invention. To implement the medical data augmentation aspect, the following steps may be implemented by the system 10 of Fig. 1 according to an embodiment of the invention:
(i) During generation of the medical data, the location of the user is identified by a real time location system (RTLS) based on a tag ID of the user. In some cases, other characteristics of the user may be captured, such as team, role, and expertise;
(ii) During generation of the medical data, the location of the medical data generation device is identified by the RTLS system based on the tag ID of the medical data generation device;
(iii) The medical data generation device sends the generated medical data (including the source, action, and data elements) to a hospital information system and location augmenter via a data protocol;
(iv) The location augmenter retrieves the location of the medical data generation device and the nearby user(s) during generation of the generated medical data from the RTLS;
(v) The location augmenter augments medical data generated by the medical data generation device with the generation distance data of nearby user(s);
(vi) The location augmenter then sends the augmented medical data to the hospital information system;
(vii) The hospital information system may further annotate the augmented medical data with user(s) information, or may simply store the augmented medical data.

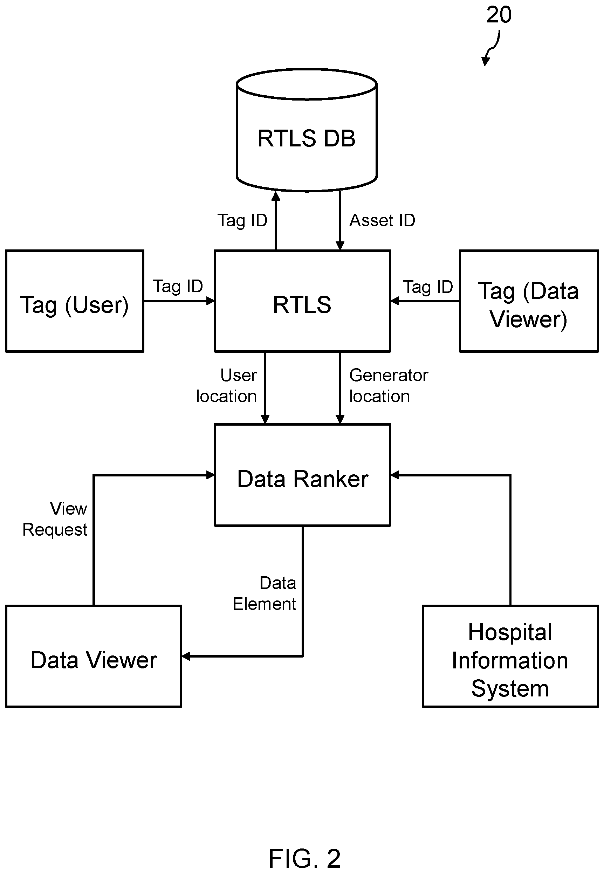

Fig 2 presents a block diagram of a system for presenting augmented medical data according to another aspect of the illustrative embodiment of the invention. To implement the usage of the system 20 and the augmented medical data, the following steps may be implemented according to an aspect of an embodiment of the invention:
(i) The location of users (i.e., potential viewers) are identified by the RTLS system based on the tag ID of the users;
(ii) The location of a device adapted to output the medical data (i.e. a data viewer) is identified by the RTLS system based on the tag ID of the data viewer;
(iii) The data viewer sends a view request to a data ranker, which may be an information broker;
(iv) The data ranker retrieves the augmented medical data from the hospital information system based on the view request;
(v) The data ranker ranks, filters, and/or clusters medical data based on the generation distance data of the augmented medical data, and based on the distance of users in proximity to the data viewer. This proximity may be refined using other characteristics of the users in proximity of the data viewer such as role, expertise, team, unit and hospital associated with the user. Furthermore, information related to the patients with which the current user is involved/associated with may also be considered (e.g., the current workflow, protocol and conditions).

For example, considering a scenario where a second nurse takes over work from a first nurse. An association of the medical data to the second nurse may be established (based on role) if the first nurse has been near the data generator for a given minimum period of time. The system will thus present medical data to the second nurse because both were associated with the medical data based on the generation distance data. In other words, as the first nurse is associated with the data due to proximity during the generation process, and the first nurse and second nurse are associated with each other (due to similar roles, responsibilities, patients, and hospital unit, for example), the medical data can be associated with the second nurse.
(vi) The data ranker sends the ranked medical image data to the data viewer.
(vii) The data viewer outputs the ranked data to the viewers(s).
Of course, the above defined systems may be refined in many ways. For one, design rules may use the measured user distance in the generation distance data to model workflow. This enables the system to provide one fluid dataflow in a workflow that is being executed by multiple users.

It may also be the case that different definitions of distance can be used. For example, different hospitals may have different definitions of distance (i.e. different definitions of proximate, distant, etc,), as well as different means and methods for calculating distance information. Since the ultimate goal of the invention is to improve access to (appropriate) medical data, and since different institutions have different means or problems with access to medical data, it is expected that their distance information is defined so that the solution contributes most to their particular use case.

For example, one hospital may decide to apply the rules for providing the medical data according to patient types and patient conditions, while another may take workflow information as basis, and another may decide on distance rules according to the data type of the medical data.

Furthermore, the above detailed system may aid clinical communication. For example, when communication is initiated, the medical data linked (via the generated distance data) to all users may be immediately made available.

It is also envisioned that the proposed system may aid in controlling an outbreak of a virus or bacteria. Starting from one user, medical data generation device or patient, the system may reconstruct potential transmissions via the augmented generation distance data. That is, it is known that person-to-person transmission plays a central role in the spread of bacteria in healthcare settings, and therefore knowledge of the distance of users during generation of data (and therefore implicitly distance between users, and distance between users and a patient) may be leveraged to further understand/track said transmission.

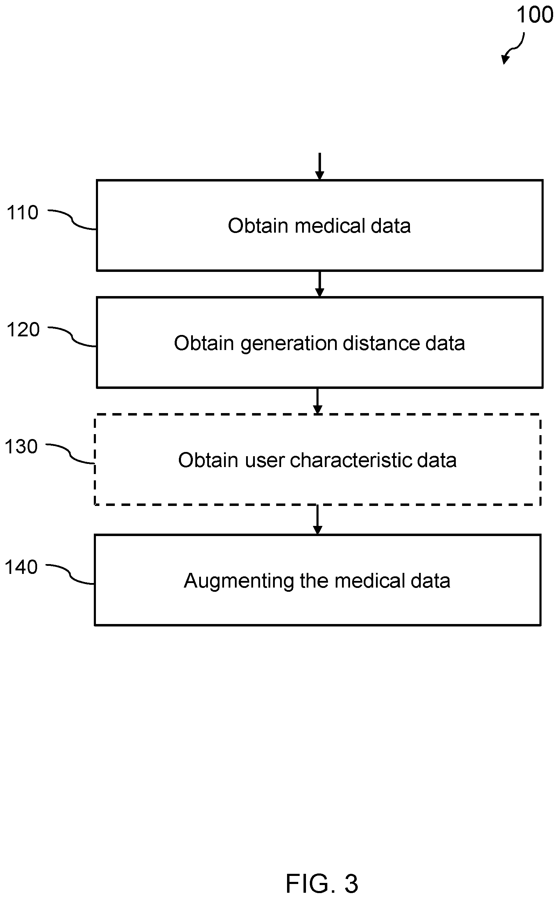

Fig.3 presents a flow diagram of a method 100 of generating augmented medical data. That is, there is provided a method of augmenting medical data generated by a data generation device.

In step 110, medical data generated by a medical data generation device is obtained. The medical data may be obtained directly from the medical data generation device, a medical treatment, or may be obtained indirectly (e.g., from an EMR system, patient information centre, data storage device, data storage system, and/or medical database). The medical data may be any data relevant to the health and/or physiological state of a subject, for example vital signs, medical images, bodily measurements, etc. The medical data may also include workflow and protocol information which guided/instructed the collection of data relevant to health and/or physiological state of the subject.

The medical data generation device may include any means for generating said medical data. For example, the medical data generation device is considered to mean hardware generating medical data, software for processing medical data to produce further medical data based on simulations/processing using a model and/or mathematical formula. Furthermore, the medical data may be generated by clinicians (e.g., a manual heart rate reading) in the form of clinician notes and observations, making the clinician a medical data generation device in certain contexts.

In step 120, generation distance data is obtained. The generation distance data describes a distance between one or more users and the medical data generation device during generation of the obtained medical data.

The one or more users may include any individual operating the medical data generation device, any observers of the generation of the medical data by the medical data generation device, and any other individuals in the vicinity of the medical data generation device. Essentially, the one or more users may include any people present while the medical data is generated. This may primarily include healthcare professionals, but may also include the subject of the medical data, and any other individuals.

The generation distance data may include any indication of the distance between the user and the device used to generate the medical data. For example, the generation distance data may include a measurement of the distance, or may be a discrete value such as near, medium and far. In addition, the generation distance data may include a distance distribution describing the distance between the user and the device for the whole time the medical data is generated, or may include a smaller number of sample distance values (e.g., an average distance, a maximum and minimum distance, a random sampling of distances during generation, etc.). In any case, the generation distance data at least provides an indication as to how close the user was to the medical data generation device during generation.

In some cases, obtaining the generation distance data may comprise determining, for each of the one or more users, a value indicative of a distance between the user and the medical data generator. This may be achieved by a signal strength that is inversely proportional to the distance between the medical data generator device and the user, or by determining the absolute location(s) of the user and device. For example, the device and user may be equipped with wireless sensors that can communicate with each other, then the distance between the device and the user can be determined based on the signal strength calculations (without determination of the location).

That is, different calculations may apply depending on the type of data collected to determine the value indicative of distance. In other words, once the distance data is collected (either in form of difference between two location points, or in terms of signal strength variations between the user and device), then this number can be further processed by taking into account the type of data to calculate a value indicative of distance.

In some cases, determining the value indicative of the distance between the user and the medical data generator may comprise obtaining a location of the user during generation of the obtained medical data, and obtaining a location of the medical data generator during generation of the obtained medical data. That is, an absolute location of the user and device is gathered. From such information, the value indicative of distance can be generated in a manner readily appreciated by the skilled person.
In specific embodiments the location of the one or more users and/or the medical data generator may be obtained from a real time location system. Thus, the locations can be acquired using a known technology.

Furthermore, the method 100 may further comprise an additional optional step of comparing the generation distance data to a predetermined requirement. The obtained medical data is then only augmented responsive to the generation distance data satisfying the predetermined requirement.

That is, there is provided a step of checking that the generation distance data meets a condition, which may include at least one of a maximum distance, and a minimum amount of time within a predetermined distance. Of course, other conditions are envisioned and would be readily appreciated by the skilled person. Such conditions may be provided/selected by a user of the method.

In optional step 130, user characteristic data describing at least one characteristic of the one or more users is obtained. The user characteristic data may include at least one of a user identifier, a user role, a user action, a user group, a user experience, and a user expertise.

The user characteristic data may be obtained automatically from a user profile database, or may be input by the user(s) during generation of the medical data. Said characteristics of the user may also be added/appended as meta data to the medical data.

The determination of the user characteristics can be based on one or more of:
(i) User identification systems based on RLTS system, where profiles for users may be stored;
(ii) User batch information, where a user ID is used to identify the user (for example, prior to medical data generation device usage);
(iii) User recognition based on image, video, or speech processing;
(iv) Prior workflow information, from which it can be estimated what types of users are usually collecting the data. For example, blood pressure measurements are performed typically by nurses, while more complicated measurements are performed by physicians;
(v) The workflow information above may be combined with user schedule and shift information; and
(vi) EMR data, where information about which clinicians are assigned to which patients is available.

In step 140, the obtained medical data is augmented based on (i.e. using) the obtained generation distance data. Augmenting the obtained medical data may comprise linking the generation distance data as metadata to the medical data. Alternatively, or additionally augmenting the obtained medical data may comprise annotating the medical data with the generation distance data. In essence, augmenting the medical data based on the obtained generation distance data means that information describing a distance between one or more users and the medical data generation device is associated with the medical data in way that when the medical data is looked up, the distance information is readily available.

Finally, if optional step 130 is provided, then the obtained medical data may also be augmented based on (i.e. using) the obtained user characteristic data. That is, the user characteristic data may be linked to, and/or used to annotate, the medical data.

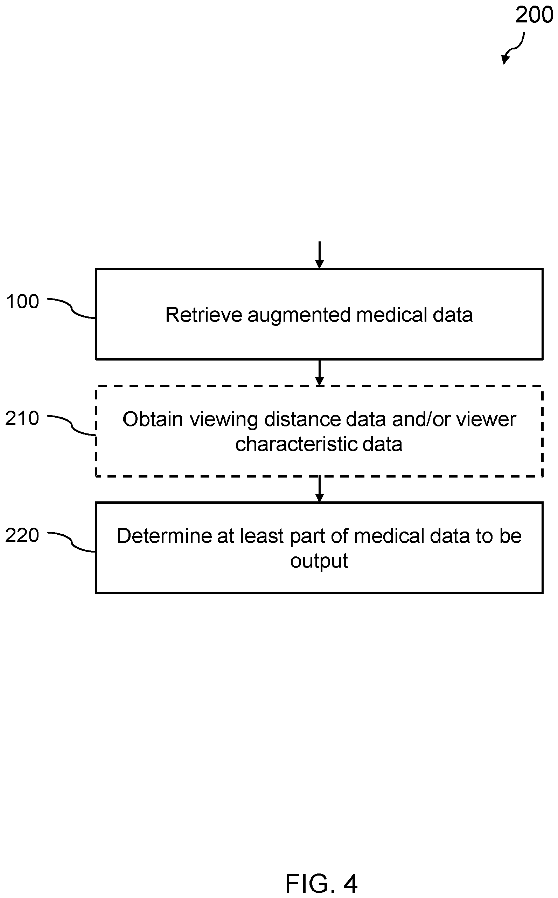

Fig.4 presents a flow diagram of a method 200 for providing medical data to a viewer according to a further aspect of the invention. Like the user as described in reference to Fig.3, the viewer may be any individual(s) that are either controlling a device configured to output the medical data, an observer, or any other individual in the vicinity of the device configured to output the medical data.
In step 100, augmented medical data (generated according to a method within the scope of method 100) is retrieved. That is, the augmented medical data may be generated, or may have been stored and then retrieved in step 100. This step may also involve extracting (using known techniques) the generation distance data from the augmented medical data, such that the medical data and associated generation distance data are obtained.

Then, in step 220, at least a part of the medical data to be output to the viewer is determined. This is achieved based on the generation distance data. That is, the parts of the medical data appropriate for output for viewing by a viewer is determined based on comparison of the generation distance data to a ruleset.

Optionally, the method may further include step 210. In step 210, viewing distance data and/or viewer characteristic data is obtained. More particularly, viewing distance data describing a distance between the viewer and a device adapted to output medical data may be obtained. This distance may be determined in the same way as the generation distance data described above. Additionally and/or alternatively, characteristic data describing at least one characteristic of the viewer may be obtained. This characteristic data may be similar to the user characteristic data described above.

In case one or both of the above are obtained, the determination of the (part of) medical data to be output to the viewer may be further based on the viewing distance data and/or viewer characteristic data as appropriate.

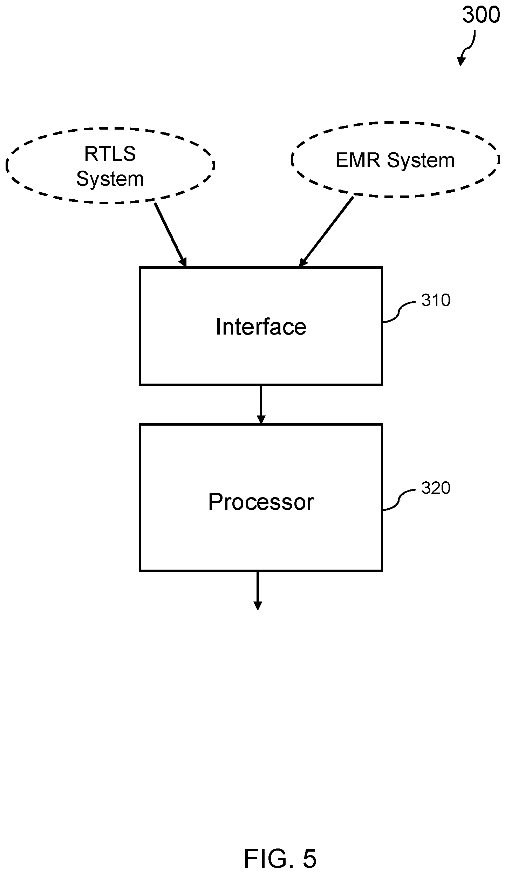

Fig.5 is a simplified block diagram of a system 300 for augmenting medical data according to yet another embodiment of the invention. The system may be implemented in multiple different devices, or on one device. The system 300 comprises an interface 310, and a processor 320.

The interface 310 is configured to obtain medical data generated by a medical data generation device, and obtain generation distance data describing a distance between one or more users and the medical data generation device during generation of the obtained medical data. Furthermore, the interface may be optionally configured to obtain user characteristic data as described above.

The generation distance data may be acquired from a RTLS, however embodiments are not restricted to such. For example, the generation distance data may be acquired from the medical data generation device itself, or another device. Also, the medical data may be acquired from an EMR system, but embodiments also aren't restricted thereto. For example, the medical data may be acquired directly from the medical data generation device.

The processor 320 is configured to augment the obtained medical data based on the obtained generation distance data. This may be achieved by any known means for editing data, whether it is implemented by an image manipulation module, or another editing platform.

Overall, the system 300 may receive the medical data and associated generation distance data, and output augmented medical data. The augmented medical data may be stored in an EMR system, hospital information system, or another database for later use (e.g., medical data analysis or output).

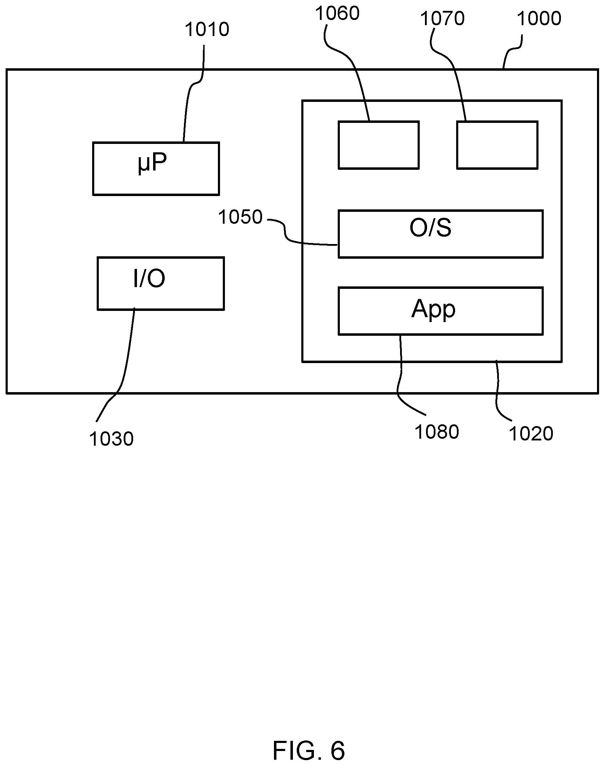

Fig.6 illustrates an example of a computer 1000 within which one or more parts of an embodiment may be employed. Various operations discussed above may utilize the capabilities of the computer 1000. For example, one or more parts of a system for acquiring an input from a user to control an interface may be incorporated in any element, module, application, and/or component discussed herein. In this regard, it is to be understood that system functional blocks can run on a single computer or may be distributed over several computers and locations (e.g. connected via internet).

The computer 1000 includes, but is not limited to, smart phones, PCs, workstations, laptops, PDAs, palm devices, servers, storages, and the like. Generally, in terms of hardware architecture, the computer 1000 may include one or more processors 1010, memory 1020 and one or more I/O devices 1030 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 1010 is a hardware device for executing software that can be stored in the memory 1020. The processor 1010 can be virtually any custom made or commercially available processor, a central processing unit (CPU), a digital signal processor (DSP), or an auxiliary processor among several processors associated with the computer 1000, and the processor 1010 may be a semiconductor-based microprocessor (in the form of a microchip) or a microprocessor.

The memory 1020 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 1020 may incorporate electronic, magnetic, optical, and/or other types of storage media. Note that the memory 1020 can have a distributed architecture, where various components are situated remote from one another, but can be accessed by the processor 1010.

The software in the memory 1020 may include one or more separate programs, each of which comprises an ordered listing of executable instructions for implementing logical functions. The software in the memory 1020 includes a suitable operating system (O/S) 1050, compiler 1060, source code 1070, and one or more applications 1080 in accordance with exemplary embodiments. As illustrated, the application 1080 comprises numerous functional components for implementing the features and operations of the exemplary embodiments. The application 1080 of the computer 1000 may represent various applications, computational units, logic, functional units, processes, operations, virtual entities, and/or modules in accordance with exemplary embodiments, but the application 1080 is not meant to be a limitation.

The operating system 1050 controls the execution of other computer programs, and provides scheduling, input-output control, file and data management, memory management, and communication control and related services. It is contemplated by the inventors that the application 1080 for implementing exemplary embodiments may be applicable on all commercially available operating systems.

Application 1080 may be a source program, executable program (object code), script, or any other entity comprising a set of instructions to be performed. When a source program, then the program is usually translated via a compiler (such as the compiler 1060), assembler, interpreter, or the like, which may or may not be included within the memory 1020, so as to operate properly in connection with the O/S 1050. Furthermore, the application 1080 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, functional programming and the like.

The I/O devices 1030 may include input devices such as, for example but not limited to, a mouse, keyboard, scanner, microphone, camera, etc. Furthermore, the I/O devices 1030 may also include output devices, for example but not limited to a printer, display, etc. Finally, the I/O devices 1030 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 1030 also include components for communicating over various networks, such as the Internet or intranet.

If the computer 1000 is a PC, workstation, intelligent device or the like, the software in the memory 1020 may further include a basic input output system (BIOS) (omitted for simplicity). The BIOS is a set of essential software routines that initialize and test hardware at start-up, start the O/S 1050, and support the transfer of data among the hardware devices. The BIOS is stored in some type of read-only-memory, such as ROM, PROM, EPROM, EEPROM or the like, so that the BIOS can be executed when the computer 800 is activated.

When the computer 1000 is in operation, the processor 1010 is configured to execute software stored within the memory 1020, to communicate data to and from the memory 1020, and to generally control operations of the computer 1000 pursuant to the software. The application 1080 and the O/S 1050 are read, in whole or in part, by the processor 1010, perhaps buffered within the processor 1010, and then executed.

When the application 1080 is implemented in software it should be noted that the application 1080 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 1080 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods described in relation to Figs.3 and 4, and the systems described in relation to Figs. 1, 2 and 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagram in Figs. 1, 2 and 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method of generating augmented medical data, comprising:
obtaining (110) medical data generated by a medical data generation device;
obtaining (120) generation distance data describing a distance between one or more users and the medical data generation device during generation of the obtained medical data; and
augmenting (140) the obtained medical data based on the obtained generation distance data.

2. The method of claim 1, further comprising:
obtaining (130) user characteristic data describing at least one characteristic of the one or more users; and
augmenting (140) the obtained medical data based on the obtained user characteristic data.

3. The method of claim 2, wherein the user characteristic data includes at least one of a user identifier, a user role, a user action, a user group, a user experience, and a user expertise.

4. The method of any of claims 1-3, wherein obtaining (120) the generation distance data comprises determining, for each of the one or more users, a value indicative of a distance between the user and the medical data generator.

5. The method of claim 4, wherein determining the value indicative of the distance between the user and the medical data generator comprises:
obtaining a location of the user during generation of the obtained medical data;
obtaining a location of the medical data generator during generation of the obtained medical data; and
generating the value based on the obtained location of the user and the obtained location of the medical data generator.

6. The method of claim 5, wherein the location of the one or more users and/or the medical data generator is obtained from a real time location system.

7. The method of any of claims 1-6, further comprising:
comparing the generation distance data to a predetermined requirement, and wherein augmenting the obtained medical data based on the obtained distance data is performed responsive to the generation distance data satisfying the predetermined requirement.

8. The method of claim 7, wherein the predetermined requirement comprises at least one of a maximum distance, a minimum amount of time within a predetermined distance, and a predefined location requirement.

9. The method of any of claims 1-8, wherein augmenting (140) the obtained medical data comprises linking the generation distance data as metadata to the medical data, and/or annotating the medical data with the generation distance data.

10. A method for providing medical data to a viewer, comprising:
retrieving augmented medical data generated (100) according to any of claims 1-9;
determining (220) at least a part of the medical data to be output to the viewer based on the generation distance data.

11. The method of claim 10, further comprising:
obtaining (210) viewing distance data describing a distance between the viewer and a device adapted to output medical data, wherein determining (220) the part of the medical data to be output to the viewer is further based on the viewing distance data.

12. The method of claim 10 or 11, further comprising:
obtaining (210) viewer characteristic data describing at least one characteristic of the viewer, and wherein determining (220) the part of the medical data to be output to the viewer is further based on the obtained viewer characteristic data.

13. A method for providing medical data to a viewer, comprising:
obtaining (110) medical data generated by a medical data generation device;
obtaining (120) generation distance data describing a distance between one or more users and the medical data generation device during generation of the obtained medical data; and
augmenting (140) the obtained medical data based on the obtained generation distance data;
retrieving the augmented medical data; and
determining (220) at least a part of the medical data to be output to the viewer based on the generation distance data.

14. A computer program comprising computer program code means adapted, when said computer program is run on a computer, to implement the method of any of claims 1-13.

15. A system for generating augmented medical data, comprising:
an interface (310) configured to:
obtain medical data generated by a medical data generation device; and
obtain generation distance data describing a distance between one or more users and the medical data generation device during generation of the obtained medical data; and
a processor (320) configured to augment the obtained medical data based on the obtained generation distance data.
